# EUROPEAN PATENT APPLICATION

(11) **EP 3 409 789 A1**
(43) Date of publication of application: **05.12.2018**
(21) Application number: 17743725.8
(22) Date of filing: 24.01.2017
(51) Int. Cl.: C12Q 1/68, C12Q 1/06, C12Q 1/04, C12Q 1/70

(54) **METHOD FOR QUALITATIVE AND QUANTITATIVE DETECTION OF MICROORGANISM IN HUMAN BODY**

(30) Priority: 29.01.2016 CN 201610061014
(71) Applicant: Jianghan University, Wuhan, Hubei 430056 (CN); Children's Hospital Medical Center, Cincinnati, OH 45229-3039 (US)
(72) Inventor: PENG, Hai, Wuhan Hubei 430056 (CN); ZHANG, Ying, Wuhan Hubei 430056 (CN); LU, Long, Wuhan Hubei 430056 (CN)
(74) Representative: Cabinet Becker et Associés
(86) International application number: PCT/CN2017/072441
(87) International publication number: WO 2017/129110

(57) **Abstract**

The present invention discloses a method for qualitative and quantitative detection of a microorganism in a human body, which belongs to the field of biotechnology. The method includes the following steps: determining a target microbial population, a target microorganism and a non-target organism in a sample to be tested, as well as a reference microorganism not present in the sample to be tested; designing the characteristic regions of the target microbial population and the target microorganism; designing multiplex amplification primers for the characteristic regions; adding the reference microorganism and an exogenous nucleic acid into the sample to be tested, and then extracting the nucleic acid of the microorganism in the sample to be tested; amplifying the nucleic acid of the microorganism with the designed multiplex amplification primers so as to obtain a characteristic sequencing fragment; and then performing, using the characteristic sequencing fragment, qualitative and quantitative analysis for the microorganism in the sample to be tested. The present invention does not need pre-culture and proliferation of the microorganism, and can perform high throughput, high accuracy and high resolution detection on a plurality of known microorganisms in the sample to be tested at one time, and the detection process is simple, quick and the process is standardized.

Sequence Listing

<110> Jianghan University; Cincinnati Children's Hospital Medical
Center)

<120> Method for qualitative and quantitative detection of microorganism in
human body

<160> 18
<170> PatentIn version 3.4

<210> 1
<211> 17
<212> DNA
<213> Artificial sequence

<400> 1
gctgttcacc gtcgcct 17

<210> 2
<211> 19
<212> DNA
<213> Artificial sequence

<400> 2
gatctccagg tagctggcc 19

<210> 3
<211> 19
<212> DNA
<213> Artificial sequence

<400> 3
accagcaact ggcgcaata 19

<210> 4
<211> 18
<212> DNA
<213> Artificial sequence

<400> 4
gctgtccagc tgcaggtt 18

<210> 5
<211> 17
<212> DNA
<213> Artificial sequence

<400> 5
cgcctggccc ttttcct 17

<210> 6
<211> 17
<212> DNA
<213> Artificial sequence

<400> 6
acgatcaggc cgtgtgc 17

<210> 7
<211> 18
<212> DNA
<213> Artificial sequence

<400> 7
ccgcgaatga gtgtcgtg 18

<210> 8
<211> 23
<212> DNA
<213> Artificial sequence

<400> 8
ccggcaacat ctctgtgata tcg 23

<210> 9
<211> 19
<212> DNA
<213> Artificial sequence

<400> 9
cggccatttg catcttcgg 19

<210> 10
<211> 22
<212> DNA
<213> Artificial sequence

<400> 10
gatttcatgg gcaatcgcct tc 22

## Description

### Technical Field

The present invention relates to the field of biotechnology, particularly to a method for qualitative and quantitative detection of a microorganism in a human body.

### Background Art

Human microorganisms are an important basis for the diagnosis of human diseases. It is necessary to accurately perform qualitative and quantitative detection of human microorganisms.

The currently available technologies of qualitative and quantitative detection of human microorganisms include morphological counting, chip detection, 16S rRNA sequencing, metagenomic sequencing and real-time quantitative PCR (Polymerase Chain Reaction). Morphological counting requires pre-culture of microorganisms, which would take a long period of time. In addition, it cannot detect the non-culturable microorganisms. Only one type of microorganism can be detected at a time, the throughput is low, and the sampling amount is limited at the time of counting. Accordingly, the obtained result is rough, and the classification unit below species cannot be distinguished. As for the chip detection, the required amount of DNA in the sample to be tested is large, and the microorganisms need to be pre-cultured and enriched, the detection result is inaccurate, and a quantitative detection cannot be performed. 16S rRNA sequencing cannot distinguish the classification unit below species. The metagenomic sequencing has a limited depth, and the accuracy of quantitative detection for low-level microorganisms is undesirable. Moreover, real-time quantitative PCR can only detect one microorganism at a time, and the throughput is low. In addition, the common drawback of existing methods is that the reliability of microbial qualitative and quantitative detection cannot be calculated, which make the obtained conclusion poor practicable. The above technical defects have caused problems such as untimely diagnosis of human diseases, inaccurate diagnosis and misdiagnosis.

### Summary of the Invention

In order to solve the problem that the microbial qualitative and quantitative detection are inaccurate in the existing technology, the embodiments of the present invention provide a qualitative and quantitative detection method for human microorganisms. The technical solution is as follows:
The present invention provides a method for qualitative and quantitative detection of a microorganism in a human body, the method includes:
determining a target microbial population, a target microorganism and a non-target organism in a sample to be tested, and a reference microorganism not present in the sample to be tested, wherein the sample to be tested is a human tissue, body fluid and feces;
obtaining a characteristic region of the target microbial population, a characteristic region of the target microorganism and a characteristic region of the reference microorganism according to the reference genomic sequences of the target microbial population, the target microorganism, the reference microorganism and the non-target organism;
preparing a first multiplex amplification primer for amplifying the characteristic region of the target microbial population, a second multiplex amplification primer for amplifying the characteristic region of the target microorganism, and a third multiplex amplification primer for amplifying the characteristic region of the reference microorganism, and mixing the first multiplex amplification primer, the second multiplex amplification primer and the third multiplex amplification primer so as to obtain mixed multiplex amplification primers;
adding the reference microorganism to the sample to be tested so as to obtain a mixed sample;
extracting the nucleic acid of the mixed sample;
carrying out an amplification reaction using the mixed multiplex amplification primers and the nucleic acid of the mixed sample, so as to obtain an amplification product;
carrying out a high throughput sequencing using the amplification product, so as to obtain a high throughput sequencing fragment; and
carrying out qualitative and quantitative analysis with the target microbial population and the target microorganism.

More specifically, the number of the target microbial population is ≥ 1, and each target microbial population contains ≥ 0 types of the target microorganism;
the target microorganism is at least one selected from the group consisting of bacterium, virus, fungus, actinomycetes, rickettsia, mycoplasma, chlamydia, spirochete and protozoa; and
the reference microorganism is at least one selected from the group consisting of bacterium, virus, fungus, actinomycetes, rickettsia, mycoplasma, chlamydia, spirochete and protozoa.

More specifically, the step of determining a non-target organism in a sample to be tested is carried out by a method that comprises: determining the non-target organism to be all organisms except the target microbial population, if the characteristic region of the target microbial population is obtained, the non-target organism referring to all organisms except the target microbial population; if the characteristic region of the target microbial population is not obtained, the non-target organism refers to the organisms other than the target microbial population in the mixed sample.

More specifically, the characteristic region of the target microbial population is a nucleic acid sequence on a reference genome of the microorganism within the target microbial population; sequences on both sides of the characteristic region of the target microbial population are a single sequence in the reference genome; the sequences on both sides of the characteristic region of the target microbial population are conservative among different microorganisms in the target microbial population; and the distinguishing degree of the characteristic region of the target microbial population is ≥ 3;
the characteristic region of the target microorganism is homologous to the characteristic region of the target microbial population; the characteristic region of the target microorganism has an m2 value ≥ 2, wherein the m2 value is a minimum value of the number of different bases between the characteristic region of the target microorganism and the microorganisms other than the target microorganism within the target microbial population;
the characteristic region of the reference microorganism is a nucleic acid sequence in the reference genome of the reference microorganism; sequences on both sides of the characteristic region of the reference microorganism are a single sequence in the reference genome of the reference microorganism; the sequences on both sides of the characteristic region do not have homology in organisms other than the reference microorganism.

Further, the distinguishing degree refers to a minimum value of the number of different bases between a characteristic region of any target microbial population and any non-characteristic region amplified by the same mixed multiplex amplification primers, wherein the non-characteristic region is an amplification product of the mixed multiplex amplification primers with the nucleic acid of the mixed sample as a template, and the non-characteristic region is not a characteristic region of the target microbial population; if the non-characteristic region is absent, the distinguishing degree is 3 × L1/4, wherein L1 is the length of a nucleic acid sequence of the characteristic region of the target microbial population.

More specifically, when extracting a nucleic acid of the mixed sample, if the content of the nucleic acid in the sample to be tested is too low, in the process of extracting the nucleic acid of the mixed sample, an exogenous nucleic acid that cannot be amplified by the mixed multiplex amplification primers is added.

More specifically, a qualitative analysis method of the target microbial population and the target microorganism is as follows:
comparing the high throughput sequencing fragment with the characteristic region of each target microbial population, and when the number of different bases is ≤ n1, the comparison is successful, and the corresponding high throughput sequencing fragment is the characteristic region of the target microbial population, wherein n1 is a maximum error-tolerant number of bases of a characteristic sequencing fragment of the target microbial population; and if the characteristic region of the target microbial population of a successful comparison ≥ 1, determining that the high throughput sequencing fragment is the characteristic sequencing fragment of the target microbial population;
comparing the characteristic region of the target microorganism with the characteristic region of each of the homologous target microbial populations, and extracting the different bases from the characteristic region of the target microorganism to form a standard genotype of the target microorganism; extracting the bases corresponding to the standard genotype of the target microorganism from the characteristic sequencing fragment of the target microbial population to form a test genotype of the target microorganism; if the number of different bases between the test genotype of the target microorganism and the standard genotype of the target microorganism ≤ n2, wherein n2 is a maximum error-tolerant number of bases of the characteristic sequencing fragment of the target microorganism, the high throughput sequencing fragment where the test genotype of the target microorganism is located is a characteristic sequencing fragment of the target microorganism;
calculating the obtained characteristic sequencing fragment of the target microorganism with the reference microorganism as the target microbial population that contains only one target microorganism, which is the characteristic sequencing fragment of the reference microorganism;
if the probability of the characteristic sequencing fragment of the target microbial population P5 ≥ α5, determining that the target microbial population is present in the sample to be tested, wherein α5 is a probability guarantee; if the probability of the characteristic sequencing fragment of the target microbial population P5 < α5, determining that the target microbial population is not present in the sample to be tested;
if the probability of the characteristic sequencing fragment of the target microorganism P6 ≥ α6, determining that the target microorganism is present in the sample to be tested, wherein α6 is a probability guarantee; if the probability of the characteristic sequencing fragment of the target microorganism P6 < α6, determining that the target microorganism is not present in the sample to be tested;
n1 allowing P1 ≤ α1, and P3 ≤ α3, wherein P1 is the probability of a false positive generated when one high throughput sequencing fragment that is not a characteristic sequencing fragment of the target microbial population is misidentified as a characteristic sequencing fragment of the target microbial population; P3 is the probability of a false negative generated when one high throughput sequencing fragment that is a characteristic sequencing fragment of the target microbial population is misidentified as not a characteristic sequencing fragment of the target microbial population; and wherein α1 and α3 are the thresholds for respective determinations;
n2 allowing P2 ≤ α2, and P4 ≤ α4, wherein P2 is the probability of a false positive generated when one high throughput sequencing fragment that is not a characteristic sequencing fragment of the target microorganism is misidentified as a characteristic sequencing fragment of the target microorganism; P4 is the probability of a false negative generated when one high throughput sequencing fragment that is a characteristic sequencing fragment of the target microorganism is misidentified as not a characteristic sequencing fragment of the target microorganism; and wherein α2 and α4 are the thresholds for respective determinations;
P5=1-BINOM.DIST(S1, S1, P1, FALSE), P6=1-BINOM.DIST(S3, S3, P2, FALSE), S1 is the median of the number of the characteristic sequencing fragments of the target microbial population of all the characteristic regions of the target microbial population; S3 is the median of the number of the characteristic sequencing fragments of the target microorganism of all the characteristic regions of the target microorganism; FALSE is a parameter value; BINOM.DIST function returns the probability of a binomial distribution.

Further, a quantitative analysis method of the target microbial population and the target microorganism is as follows:
the amount of the target microbial population M1=Mr×S1/S2, and the confidence interval of the amount of the target microbial population is [M11, M12], wherein Mr is the amount of the reference microorganism added to the sample to be tested; S2 is the median of the number of the characteristic sequencing fragments of the reference microorganism of all the characteristic regions of the reference microorganism; M11 and M12 are respectively the lower limit and the upper limit of the confidence interval of the M1 value;
the amount of the target microorganism M2=M1×S3/S1, the confidence interval of the amount of the target microorganism is [M21, M22], and M21 and M22 are respectively the lower limit and the upper limit of the confidence interval of the M2 value;
M11=M1×(1-S4/S1), M12=M1×(1+S5/S1), M21=M2×(1-S6/S3), M22=M2×(1+S7/S3); wherein S4 is the number of the false positive characteristic sequencing fragments of the target microbial population and S4=CRITBINOM(nS,P1,α9), wherein nS is the number of the high throughput sequencing fragments of the non-characterized region amplified by the multiplex amplification primers of the characteristic region of the target microbial population for calculating S1; S5 is the number of the false negative characteristic sequencing fragments of the target microbial population and S5=CRITBINOM(S1, P3, α9), wherein α9 is a probability guarantee; S6 is the number of the false positive characteristic sequencing fragments of the target microorganism and S6=CRITBINOM(S1, P2, α10), S7 is the number of the false negative characteristic sequencing fragments of the target microorganism and S7= CRITBINOM(S3, P4, α10), where α10 is a probability guarantee; the CRITBINOM function returns a minimum value that makes a cumulative binomial distribution greater than or equal to a critical value.

Further, P1=BINOM.DIST(n1,m1,1-E,TRUE), P2=BINOM.DIST(n2,m2,1-E,TRUE), P3=1-BINOM.DIST(n1,L1,E,TRUE), and P4=1-BINOM.DIST(n2,L2,E,TRUE), wherein m1 is the distinguishing degree; m2 is a minimum value of the different bases between the characteristic region of the target microorganism and the other microorganisms within the target microbial population; L1 is the length of the characteristic region of the target microbial population; L2 is the length of the standard genotype of the target microorganism; and E is a base error rate.

The technical solutions provided by the embodiments of the present invention have the following beneficial effects: the method provided by the invention does not need to pre-culture and proliferate the microorganisms, can be finished in a short time period, can simultaneously detect a plurality of microorganisms, has high throughput, and has a large sampling amount when counting. The detection result is fine, and the classification units can be distinguished. It does not need a large amount of DNA and can avoid the enrichment culture, the detection structure is noiseless and accurate, the quantitative accuracy for low-level microorganisms is high, and the detection qualitative and quantitative test results for microorganisms are accurate. It has high resolution, high sensitivity, and probabilistic guarantee. The detection process is simple, fast and the process is standardized. The method provided by the present invention can facilitate timely and accurate diagnosis of blood diseases.

### Description of Embodiments

In order to make the objects, technical solutions and advantages of the present invention more clear, the embodiments of the present invention will be further described in detail below. The reagents not described in the present invention are commonly used, commercially available reagents, which can be purchased from different biotechnology companies, and the results obtained from them have almost no difference.

### Example 1: Identification of human blood microorganisms

The sample to be tested is a human tissue, body fluid and feces. Blood microorganisms are the basis for the diagnosis and treatment of many human diseases. The sample to be tested in the present embodiment is human blood, and is taken from a patient who is diagnosed by a doctor as having a bacteremia disease; detecting the microorganism in the blood can provide a basis for the treatment plan.

Step I - Determining a target microbial population, a target microorganism and a non-target organism in a sample to be tested, and a reference microorganism not present in the sample to be tested, and the specific method is as follows:
the number of the target microbial population is ≥ 1, and each target microbial population comprises ≥ 0 types of the target microorganism; the target microorganism is at least one selected from the group consisting of bacterium, virus, fungus, actinomycetes, rickettsia, mycoplasma, chlamydia, spirochete and protozoa. The aim of this example is to identify *Pseudomonas aeruginosa* in the sample to be tested, which has a Latin name of *Pseudomonas aeruginosa.* According to the information available on the NCBI (National Center for Biotechnology Information), there are 30 physiological races of *Pseudomonas aeruginosa* with known reference genome (up to the date of June 2, 2015); for more information, please see http://www.ncbi.nlm.nih.gov/genome/genomegroups/187. These physiological races constitute the target microbial population of this embodiment. Among these physiological races, *Pseudomonas aeruginosa* PA7 is highly pathogenic and serves as a target microorganism of the present example.

The reference microorganism is at least one selected from the group consisting of bacterium, virus, fungus, actinomycetes, rickettsia, mycoplasma, chlamydia, spirochete and protozoa. The reference microorganism is not present in the sample to be tested. The role of the reference microorganism is to provide a reference for the quantification of the target microbial population and the target microorganism in the sample to be tested. Since *Agrobacterium tumefaciens* is present in the root of a plant, it is not present in the sample to be tested. Therefore, in the present example, *Agrobacterium tumefaciens* is selected to serve as a reference microorganism, and its Latin name is *Agrobacterium tumefaciens* K84.

More specifically, the process of determining a non-target organism in a sample to be tested includes: determining the non-target organism to be all organisms except the target microbial population, if the characteristic region of the target microbial population can be obtained, the non-target organism referring to all organisms except the target microbial population; in this regard, all organisms refer to the organisms that have the reference genome, which is the most stringent criteria for the non-target organism. In this embodiment, when the non-target organism is determined to be all known organisms other than the target microbial population, the characteristic regions of the target microbial population can be found (see the process of obtaining the characteristic region below, and the results are shown in Table 1). Therefore, the non-target organism in this example is the set of all organisms except the target microbial population.

The non-target organism is determined to be all organisms except the target microbial population, if the characteristic region of the target microbial population is not obtained, the non-target organism referring to the organisms other than the target microbial population in the mixed sample, so as to narrow the range of the non-target organism and increase the likelihood of finding the characteristic region of the target microbial population. In the mixed sample, the other organisms other than the target microbial population can be determined empirically by experience. For example, in the present embodiment, the mixed sample includes blood and reference microorganisms, accordingly it is impossible to have plant components and the microorganism that superficially lives in plants. As a result, in the case that the non-target organism in this embodiment is identified as all known organisms other than the target microbial population, if the characteristic region of the target microorganisms cannot be obtained, the non-target microorganisms can be determined to be the set of organisms other than the target microorganism, plants, and the microorganisms specifically live in plants.

Step II - Obtaining a characteristic region of the target microbial population, a characteristic region of the target microorganism and a characteristic region of the reference microorganism according to the reference genomic sequence of the target microbial population, the reference genomic sequence of the target microorganism, the reference genomic sequence of the reference microorganism and the reference genomic sequence of the non-target organism:
The characteristic region of the target microbial population is a nucleic acid sequence of a reference genome of the microorganism within the target microbial population; sequences on both sides of the characteristic region of the target microbial population are a single sequence in the reference genome; the sequences on both sides of the characteristic region of the target microbial population are conservative among different microorganisms in the target microbial population; and the distinguishing degree of the characteristic region of the target microbial population is ≥ 3. The non-characteristic region is not the characteristic region of the target microbial population, the non-characteristic region is an amplification product of the mixed multiplex amplification primers with the nucleic acid of the mixed sample as a template. The distinguishing degree refers to the minimum value of the number of different bases between the characteristic region of any target microbial population amplified by the same mixed multiplex primer and any non-characteristic region. In addition, if the non-characteristic region is absent, the distinguishing degree is 3 × L1/4, wherein L1 is the length of a nucleic acid sequence of the characteristic region of the target microbial population.

More specifically, the characteristic region of the target microbial population is used to represent the target microbial population, and if the characteristic region of the target microbial population exists, it represents the existence of the target microbial population. In addition, the number of the sequencing fragments of the characteristic region of the target microbial population represents the number of the target microbial population. The ideal multiple primers of the characteristic region of the target microbial population only amplify the characteristic region of the target microbial population and do not amplify non-target organisms. This requires that the sequences on two sides of the characteristic region of the target microbial population, that is, the primer design regions, are not homologous in the non-target organisms, and in this way, the non-target organisms cannot be amplified, nor can a non-characteristic region be generated. At this time, the same base can be randomly generated between the characteristic region and the non-characteristic region. Since there are 4 kinds of bases, and the probabilities of the same base and different base are 1/4 and 3/4, respectively, the distinguishing degree is 3x L1/4. The requirement that the distinguishing degree of the characteristic region of the target microbial population is ≥ 3 is to ensure that the false positive rate and the false negative rate determined by the characteristic sequencing fragment of the target microbial population are low, and the principle is shown in Table 2. In addition, if the sequences on both sides of the characteristic region of the target microbial population are conservative among different microorganisms in the target microbial population, the same primers can be used to amplify different microorganisms in the target microbial population so as to eliminate the influence of amplification efficiency on the relative quantification among different microorganisms in the target microbial population.

The characteristic region of the target microorganism is homologous to the characteristic region of the target microbial population; the characteristic region of the target microorganism has an m2 value ≥ 2, wherein the m2 value is a minimum value of the number of different bases between the characteristic region of the target microorganism and the microorganisms other than the target microorganism within the target microbial population. In this embodiment, the other microorganisms refer to the physiological races in target microbial population other than the target microorganism, and the m2 value is the minimum value of the number of the different bases obtained when comparing the characteristic region of the target microorganism with the homologous regions of other physiological races in the target microbial population. In the qualitative and quantitative analysis of a target microorganism, the focus is on distinguishing it from other microorganisms in the target microbial population. The target microorganism is usually closely related to the target microbial population, and the similarity between their sequences is high, so it is difficult to distinguish them. In the qualitative and quantitative analysis of the target microorganism, only the standard genotypes in the amplicon which are different from other microorganisms in the target microbial population are concerned, which reduces the potential source of the error, so that the target microorganism can be better separated from the target microbial population. When m2 ≥ 2, the false positive rate and the false negative rate are low for determining whether the sequencing fragment is the characteristic sequencing fragment of the target microorganism; therefore, the target microorganism can be distinguished from the target microbial population, and the principle thereof is shown in Table 2.

The characteristic region of the reference microorganism is a nucleic acid sequence in the reference genome of the reference microorganism; sequences on both sides of the characteristic region of the reference microorganism are a single sequence in the reference genome of the reference microorganism; the sequences on both sides of the characteristic region do not have homology in organisms other than the reference microorganism.

In this embodiment, the distinguishing degree is the only selection criterion for the characteristic region of the target microbial population, and depending on the purpose of the detection, the microorganism having a specific gene sequence may be used as the target microbial population, and the specific gene sequence is taken as the characteristic region of the target microbial population. For example, the microorganism having a specific pathogenic gene can be used as the target microbial population, and the pathogenic gene can be used as the characteristic region of the target microorganism so as to guide the drug treatment according to the type of the pathogenic gene. Similarly, a drug-resistant gene can also be used as a specific gene sequence to guide drug treatment.

Step III - Prepare a first multiplex amplification primer for amplifying the characteristic region of the target microbial population, a second multiplex amplification primer for amplifying the characteristic region of the target microorganism, and a third multiplex amplification primer for amplifying the characteristic region of the reference microorganism, and mixing the first multiplex amplification primer, the second multiplex amplification primer and the third multiplex amplification primer so as to obtain mixed multiplex amplification primers.

The specific method combining step II and step III is as follows:
The genomic sequences of various physiological races within the target microbial population were downloaded from ftp://ftp.ncbi.nlm.nih.gov/genomes/ and their genomes are compared with the query sequence (reference sequence) for analysis with the software Megablast (version 2.2.26). In this example, the query sequence is the genomic sequence with the accession number AE004091 from NCBI. The parameters of the Megablast software comparison are set to as follows: parameter -e is set to 1e-5; parameter -p is set to 0; parameter -v is set to 5000; parameter -m is set to 1. After the comparison or alignment is completed, homologous sequences among all microorganisms of the target microbial population are obtained, and the homologous sequence(s) that appear(s) only once in the query sequence are further selected. With a window at the size of 110 bp and a step at the size of 10 bp, a window translation process is performed within the selected homologous sequence(s). For each window obtained by translation, compare the bases that differ between at least two microorganisms in the target microbial population, and select the region from the first different base to the last different base in the window to be the characteristic region, and then count the number of different bases in that characteristic region. A region extending for a length of 160 bp from each of the two sides of the characteristic region is used as a primer search region, and within the primer search region search for the region that has a length greater than 20 bp and has no base difference among all microorganisms in the target microbial population, which will be used as the primer design area of the characteristic region, while the characteristic region lacking such primer design area will be discarded.

Log in to the multiplex primer online design page at https://ampliseq.com and then select "DNA Hotspot designs (single-pool)" under the option of "Application type." If the multi-pool is selected in this example, the multiplex PCR will be performed in multiple tubes, and the cost will increase. On the contrary, for the selection of single-pool primers, it only requires one multiplex PCR, which can save the costs, but the disadvantage would be that the primer design of some characteristic region may fail. However, due to the large number of characteristic regions on the genome, a failure in the design of the primers of a few characteristic regions will not have significant impact on the result. In this regard, in this example, the single-pool is selected. The characteristic regions of all the target microbial populations obtained above and their corresponding primer design regions are connected by 100 bases N (N represents any one of the four bases A, T, C and G) so as to generate a reference genome for primer design. After selecting "Custom" under the option of "Select the genome you wish to use," the generated reference genome for primer design is uploaded, and then select "Standard DNA" under the option of "DNA Type." Next, in the "Add Hotspot" option, fill in the start and end positions of the characteristic region in the generated reference genome for primer design. Finally, click the button "Submit targets" to submit and obtain the multiplex primer sequences of the characteristic regions of the target microbial population.

Next, use the designed multiplex primers to carry out alignment and analysis for the target microbial population by means of BLASTN (Basic Local Alignment Search Tool) (version 2.2.26), and the forward and reverse primers, that at least one of them that has specificity is selected. The selected primers are then subjected to BLASTN alignment and analysis with the genome of the non-target organism to check whether they can amplify the genome of the non-target organism. In this example, the non-target organism refers to all of the organisms except the target microbial population, and the non-target organism's genome is NCBI's NT/NR library. The criteria for determining the amplification of the primers are as follows: the length of the amplified region is no more than 200 bp, the length of the primer matching is greater than 15 bp, and there are no base deletions or mismatches within 5 bases from the 3' end of the primer. If the primer cannot amplify any non-target organism, the characteristic region of the target microorganism corresponding to the primer has a distinguishing degree of m1=3×L1/4. If the primer can amplify a part of the non-target organisms, the amplification product of any non-target organism amplified with the primer will be compared with the characteristic region of any target microbial population, and in all the comparisons, the minimum number of different bases is the distinguishing degree m1, and the characteristic region of the target microbial population with m1 ≥ 3 will be retained, and then the characteristic region(s) containing simple repeat sequences or multiple copies in the genome will be further removed. Next, from the characteristic regions of the retained target microbial population, the characteristic regions of the target microbial population are further refined and the characteristic regions of the target microorganism are also selected.

Further, the method for refining the characteristic region of the target microbial population is as follows: the characteristic region is compared with the reference genome of the non-target organism by BLASTN, the characteristic region having more than 95% homology with the non-target organism is removed, and the remaining characteristic regions are used to compare between the target microorganisms and other microorganisms within the target microbial population using the software (version: V3.6) using the software's default parameters, so as to obtain the minimum value of the number of different bases, that is, the m2 value. The characteristic regions of the target microbial population with m2 ≥ 2 will be retained, and two or more than two of the characteristic regions with large distinguishing degrees m1 and m2 values will be selected from the retained characteristic regions to be the characteristic regions of the target microbial population and the characteristic regions of the target microorganism, while the corresponding multiplex primers will serve as the first multiplex amplification primer and the second multiplex amplification primer.

The characteristic regions of the reference microorganism and the corresponding third multiplex amplification primer are obtained in a similar manner to the method of searching for the characteristic region of the target microbial population. The following description will focus on the differences between them, while the same areas will not be repeatedly described herein. The reference microorganism genome is also aligned with the query sequence (reference sequence) using the software Megablast (version 2.2.26), in which the query sequence is the genomic sequence of *Agrobacterium tumefaciens* K84. After the alignment is completed, a single sequence in the reference microorganism genome that appears only once in the query sequence is obtained. The single sequence is then aligned with the NT/NR library of NCBI, and the single sequence with homologous sequences in the non-target organism will be further discarded. The non-overlapping length of 110 bp is randomly selected from the single sequence as the characteristic region, and the sequences on both sides thereof are also selected as the primer design region. The multiplex primers of the characteristic regions are next designed using the multiplex primer online design website https://ampliseq.com, so as to further screen the successfully designed characteristic regions of the multiplex primers. The specific method is as follows: the characteristic regions containing simple repeat sequences or having multiple copies in the genome will be removed, and the remaining characteristic regions are further compared with the reference genome of the non-target organism by BLASTN, and the characteristic regions having more than 95% homology with the non-target organism are also removed. Next, two or more characteristic regions are randomly selected from the remaining feature regions to be the characteristic regions of the reference microbial population, and the corresponding multiplex amplification primers are used as the third multiplex amplification primers.

Each one of the first multiplex amplification primer, the second multiplex amplification primer and the third multiplex amplification primer obtained in the above process, the template sequences corresponding to the amplification from each of the multiplex amplification primers, in which the template sequences refer to the amplified regions filled in the "Add Hotspot" option of each multiplex amplification primer, are synthesized by Sango Biotechnology (Shanghai) Co., Ltd. The amplification efficiency of each multiplex primer has been checked according to the operation manual of the StepOne Real-Time PCR (Part Number 4376784 Rev. E) from Thermo Fisher Scientific, Inc., and only the multiplex amplification primer with the amplification efficiency between 95% and 105% is retained, so as to reduce the impact from the differences in amplification efficiency on the qualitative and quantitative analysis for the microorganisms. Since the impact from the amplification efficiency is not significant, the characteristic region of the target microbial population and the characteristic region of the target microorganism can be different, so that it will be easier to separately find the respective characteristic regions of them. The multiplex amplification primers retained for the first multiplex amplification primer, the second multiplex amplification primer and the third multiplex amplification primer are next combined together using the combination software available on the multiplex amplification primer online design website https://ampliseq.com, so as to obtain the mixed multiplex amplification primers. The mixed multiplex amplification primers are then synthesized by the American Thermo Fisher Scientific Corporation, which are provided by the company in a liquid form. The related information for the characteristic region finally obtained in this example is shown in Table 1. The start and end positions shown in Table 1 refer to the start and end positions on the reference genome of the characteristic region on the query sequence.

**Table 1. Related information of the primers provided in the first embodiment of the present invention**

| Characteristic region | | Start position | End position | Length (L) | Upstream primer | Downstream primer | m1 value | m2 value | Number of characteristic sequencing fragments | |
|---|---|---|---|---|---|---|---|---|---|---|
| | | | | | | | | | Target microbial population | Target microorganism |
| Target | 1 | 1524 | 1524 | 20 | As | As shown | 27 | 9 | 300 | 261 |
| microbial population and target microorganism | | 076 | 281 | 6 | shown in SEQ ID No: 1 | in SEQ ID No: 2 | | | 756 | 212 |
| | 2 | 5318 646 | 5318 840 | 19 5 | As shown in SEQ ID No: 3 | As shown in SEQ ID No: 4 | 33 | 7 | 325 564 | 287 335 |
| | 3 | 3053 853 | 3054 048 | 19 6 | As shown in SEQ ID No: 5 | As shown in SEQ ID No: 6 | 14 6 | 8 | 453 345 | 350 123 |
| Reference microorganism | 1 | 1403 03 | 1404 38 | 13 5 | As shown in SEQ ID No: 7 | As shown in SEQ ID No: 8 | 13 5 | | 180376 | |
| | 2 | 1425 12 | 1426 53 | 14 1 | As shown in SEQ ID No: 9 | As shown in SEQ ID No: 10 | 14 1 | | 226777 | |
| | 3 | 5223 | 5384 | 16 1 | As shown in SEQ ID No: 11 | As shown in SEQ ID No: 12 | 16 1 | | 250689 | |

Step IV - Adding the reference microorganism to the sample to be tested so as to obtain a mixed sample, and the specific method is as follows:
The reference microorganism is not present in the sample to be tested, so the reference microorganism can be used as an internal reference and operated in parallel with the microorganism in the sample to be tested, so that the target microbial population and the target microorganism in the sample to be tested can be quantified. The amount of the reference microorganism added is controlled as can extract about 10 ng of nucleic acid (DNA) from the mixed sample so as to construct a high throughput sequencing library in a normal way, at the same time, the amount of the reference microorganism to be added should not make the proportion of reference microorganism too large, which may occupy an excessive amount of high throughput sequencing data. The method for obtaining the mixed sample in the present embodiment is as follows: 0.2 mL of bacterial solution of the reference microorganism with a concentration of 2 OD (OD is the maximum absorbance value of the bacterial solution) is loaded in a 1.5 mL centrifuge tube, which is dried by vacuum-frozen centrifugation, and then added to the sample to be tested, mix well, so as to obtain a mixed sample of the sample to be tested and the reference microorganism. The amount of the reference microorganism added to the the mixed sample is counted by an approach of blood plate counting, and the result is shown in Table 2.

Step V - Extracting the nucleic acid from the mixed sample, and the specific method is as follows:
When extracting the nucleic acid from the mixed sample, if the content of the nucleic acid in the sample to be tested is too low (less than 1 µg), it will affect the extraction effect of the nucleic acid from the mixed sample, in such a case, an exogenous nucleic acid that cannot be amplified by the multiplex amplification primers may be added during the process of extracting the nucleic acid from the mixed sample, in which the added exogenous nucleic acid does not exist in nature and thus does not interfere with the detection on microorganism. The External RNA Control Association has designed and validated a set of nucleic acid sequences that are not found in nature and can be used as exogenous nucleic acids in the examples of the present invention. The sequence can be found at https://tools.lifetechnologies.com/content/sfs /manuals/cms_095047.txt. The amount of the exogenous nucleic acid added is about 1 µg, which can ensure that the nucleic acid in the mixed sample can be extracted in a normal way. In the present embodiment, the sample to be tested is blood, its nucleic acid content is normal, and therefore, it is not necessary to add an exogenous nucleic acid to the mixed sample. The nucleic acid of the obtained mixed sample is extracted using a blood genomic DNA extraction kit (manufacturing company: Tiangen Biochemical Technology (Beijing) Co., Ltd., product number: DP348) according to the method provided in the operation manual.

Step VI - The amplification reaction is carried out using the mixed multiplex amplification primer and the nucleic acid from the mixed sample to obtain an amplification product, and the specific method is as follows:
After the nucleic acid from the mixed sample is amplified in multiplex PCR amplification using the Library Construction Kit 2.0 (manufactured by the U.S. company LifeTechnology, Inc., Cat. No. 4475345), a high throughput sequencing library is constructed using the obtained amplification product. The kit includes the following reagents: 5 x Ion AmpliSeq™ HiFi Mix, FuPa reagent, conversion reagent, sequencing adaptor solution, and DNA ligase. The process of library construction is carried out in accordance with the kit's instruction "Ion AmpliSeq™ Library Preparation" (publication number: MAN0006735, version: A.0). The amplification system of multiplex PCR is as follows: 5 × Ion AmpliSeq™ HiFi Mix 4 µl, synthetic mixed multiplex amplification primer 4 µl, extracted mixed sample nucleic acid 10 ng, and enzyme-free water 11 µl. The amplification procedure for multiplex PCR is as follows: 99°C, 2 minutes; (99°C, 15 seconds; 60°C, 4 minutes) × 25 cycles; incubation at 10°C. The excessive primers in the multiplex PCR amplification product are then digested by the FuPa reagent, and then a phosphorylation process is carried out, and the specific method is as follows: 2 µL of FuPa reagent is added to the amplification product of the multiplex PCR, and after mixing, the following procedure is performed on a PCR instrument: 50°C, 10 minutes; 55°C, 10 minutes; 60°C, 10 minutes; and saved at 10°C, so as to obtain a mixture a, where the mixture a is a solution containing a phosphorylated amplification product. The phosphorylated amplification product is linked to the sequencing adaptor by adding 4 µL of the conversion reagent, 2 µL of the sequencing adaptor solution and 2 µL of the DNA ligase to the mixture a, and after mixing, the reaction is carried out on the PCR instrument as follows: 22°C, 30 min; 72°C, 10 min; and saved at 10°C to obtain a mixture b. The mixture b is then purified by a standard ethanol precipitation method and then dissolved in 10 µL of enzyme-free water. Using the Qubit® dsDNA HS Assay Kit (Cat. No. Q32852) manufactured by the U.S. company Invitrigen to perform the assay according to the manufacturer's instructions, the mass concentration of the mixture b is obtained, and the purified mixture b is then diluted to 15 ng/ml, so as to obtain a high throughput sequencing library at a concentration of about 100 pM.

Step VII - High throughput sequencing is carried out using the amplification product to obtain high throughput sequencing fragment, and the specific method is as follows:
The obtained high throughput sequencing library and the kit Ion PI Template OT2 200 Kit v2 (manufactured by the U.S. company Invirtrigen, Cat. No. 4485146) are used to carry out an ePCR (Emulsion PCR, emulsion polymerase chain reaction) amplification before sequencing, and the process is carried out according to the manufacturer's instructions for the kit. Next, the resulting ePCR product and the kit Ion PI Sequencing 200 Kit v2 (manufactured by the U.S. company Invirtrigen, Cat. No. 4485149) are used to carry out a high throughput sequencing process on a Proton II high throughput sequencer according to the manufacturer's instructions for the kit. In this example, the amount of the high throughput sequencing is set to 1 M sequencing fragment (1 M = 1 million).

The high throughput sequencing fragments are aligned to the characteristic region of the corresponding target microbial population, the characteristic region of the target microorganism and the characteristic region of the reference microorganism according to the primers of the sequenced fragments, so as to remove the sequencing fragments that have either unsuccessful alignment or incomplete characteristic region, in which most of the sequencing fragments that have unsuccessful alignment are non-specific amplification products, while the sequencing fragments of incomplete characteristic region refers to the sequencing fragments that cannot completely detect the start position and end position of the characteristic region shown in Table 1.

Step VIII - Qualitative and quantitative analysis of the target microbial population and the target microorganism is carried out on the basis of the high throughput sequencing fragments, and the specific method is as follows:
The basic mechanism of the qualitative and quantitative analysis of a microorganism provided by the present invention is as follows: the characteristic regions represents the target microbial population and the target microorganism, and if there are sequencing fragments of the characteristic region, the target microbial population or the target microorganism exists, and the number of sequencing fragments of the characteristic region also represents the number of the target microbial population and the number of the target microorganism. Unlike other microorganism qualitative and quantitative tests, the embodiments of the present invention calculate the reliability of the microorganism qualitative and quantitative method, and at the same time, enhance the practicability of the obtained conclusion. The embodiments of the present invention need to clarify the complex relationship between the parameters and then achieve the qualitative and quantitative detection of any microorganism, and obtain a reliable conclusion. The specific parameters of the present invention and the calculation principle thereof are shown in Table 2. The definitions for the cells, symbols and formulas in Table 2 are the same as those of Excel 2010, in which the cell "basic parameter" is A1, and other cells are defined with reference to A1 according to the rules of Excel 2010.

The qualitative analysis method is as follows: compare the high throughput sequencing fragment with the characteristic region of each target microbial population, and when the number of different bases is ≤ n1, the comparison is successful, and the corresponding high throughput sequencing fragment is the characteristic region of the target microbial population, wherein n1 is a maximum error-tolerant number of bases of a characteristic sequencing fragment of the target microbial population; and if the characteristic region of the target microbial population of a successful comparison ≥ 1, determine that the high throughput sequencing fragment is the characteristic sequencing fragment of the target microbial population.

Compare the characteristic region of the target microorganism with the characteristic region of each of the homologous target microbial populations, and extract the different bases from the characteristic region of the target microorganism to form a standard genotype of the target microorganism, in which the different base refers to the sum of the different bases of the characteristic region of the target microorganism compared with any of the microorganisms in the target microbial population. Also, extract the bases corresponding to the standard genotype of the target microorganism from the characteristic sequencing fragment of the target microbial population to form a test genotype of the target microorganism; if the number of different bases between the test genotype of the target microorganism and the standard genotype of the target microorganism ≤ n2, wherein n2 is a maximum error-tolerant number of bases of the characteristic sequencing fragment of the target microorganism, the high throughput sequencing fragment where the test genotype of the target microorganism is located is a characteristic sequencing fragment of the target microorganism. In particular, in the case when only one target microorganism is contained in the target microbial population, the number of bases of the standard genotype and the test genotype is zero, and therefore, the number of different bases between them is also zero. In this case, regardless of the size of n2, the high throughput sequencing fragment of the test genotype of the target microorganism is determined to be the characteristic sequencing fragment of the target microorganism. According to the above method, the number of characteristic fragments of the target microbial population and the number of the characteristic region of the target microorganism are obtained, and the results are shown in Table 1. In the present embodiment, the values of n1 and n2 are shown in Table 2, and the calculation process will be described below.

n1 allows P1 ≤ α1, and P3 ≤ α3, wherein P1 is the probability of a false positive generated when one high throughput sequencing fragment that is not a characteristic sequencing fragment of the target microbial population is misidentified as a characteristic sequencing fragment of the target microbial population; P3 is the probability of a false negative generated when one high throughput sequencing fragment that is a characteristic sequencing fragment of the target microbial population is misidentified as not a characteristic sequencing fragment of the target microbial population; and wherein α1 and α3 are the thresholds for respective determinations.

n2 allows P2 ≤ α2, and P4 ≤ α4, wherein P2 is the probability of a false positive generated when one high throughput sequencing fragment that is not a characteristic sequencing fragment of the target microorganism is misidentified as a characteristic sequencing fragment of the target microorganism; P4 is the probability of a false negative generated when one high throughput sequencing fragment that is a characteristic sequencing fragment of the target microorganism is misidentified as not a characteristic sequencing fragment of the target microorganism; and wherein α2 and α4 are the thresholds for respective determinations. The size of various thresholds in the embodiments of the present invention is determined by actual needs. For example, some germs are extremely harmful, and missed detection (false negatives) will cause serious consequences. In this case, it is necessary to control false negatives, and accordingly, the α2 and α4 values should be low. However, in the case that there is no special requirement, the false positive rate and false negative rate should be low. This embodiment of the present invention belongs to the latter. The values of α1 and α3 are 0.01%, that is, there are 1 false positive or false negative in about 10,000 characteristic sequences. The accuracy is very high. The reason why such high accuracy needs to be controlled is because the m1 value in the characteristic sequence is large, which makes that it can be easily distinguished from other non-target organisms, thus controlling the false positive rate and the false negative rate to a very low level. The values of α2 and α4 are 0.5%, that is, there are 5 false positives or false negatives in about 1,000 characteristic sequences, which shows that the accuracy is high. P1=BINOM.DIST(n1,m1,1-E,TRUE), P2=BINOM.DIST(n2,m2,1-E,TRUE), P3=1-BINOM.DIST(n1,L1,E,TRUE), and P4=1-BINOM.DIST(n2,L2,E,TRUE), in which m1 is the distinguishing degree, and specifically refers to the distinguishing degree corresponding to the calculation of the characteristic region of the target microbial population of S1. In this embodiment, the value of m1 is shown in Tables 1 and 2; m2 is the minimum value of the number of different bases between the characteristic region of the target microorganism and the microorganisms other than the target microorganism within the target microbial population, which specifically refers to the m2 value used for calculating the characteristic region corresponding to the target microorganism of S3. In this embodiment, the value of m2 is shown in Tables 1 and 2. L1 is the length of the characteristic region of the target microbial population. In this embodiment, the value of L1 is shown in Table 2. L2 is the length of the standard genotype of the target microorganism. In this embodiment, the value of L2 is shown in Table 2. E is the base error rate, which is composed of a sequencing error rate E1 and a natural mutation rate E2. In this embodiment, the sequencing error rate of the PROTON high throughput sequencer is E1 ≤ 1%. According to our investigation, the mutation rate of the reference genomes of microbial races (such as P1-P6 blight races) is typically less than 0.5%, while the natural mutation rate is lower than the mutation rate between the races, therefore, the natural mutation rate E2 ≤ 0.5%. In order to make the present invention have broad applications, the value of E2 is selected to be ≤ 1%. Accordingly, in this embodiment, E is ≤ 2%. In order to make the probability of the accuracy of the qualitative and quantitative conclusion of the microorganism in this embodiment more reliable, the maximum value of E, that is 2%, is selected for the calculation. After substituting the above parameter values into the formulas of P1 and P3, the value of n1 is gradually increased from 0, and the values of P1 and P3 are calculated. When n1=13, it can be obtained from the calculation that P1 ≤ α1 and P3 ≤ α3. Therefore, in this embodiment of the present invention, n1 = 13 (see Table 2), and the values of P1 and P3 corresponding to n1 = 13 are the values of P1 and P3 in the present embodiment. In a similar way, after substituting the above parameter values into the formulas of P2 and P4, the value of n2 is gradually increased from 0, and the values of P2 and P4 are calculated. When n2=2, P2 ≤ α2, P4 ≤ α4. Therefore, in the present embodiment, n2 = 2 (see Table 2), and the values of P2 and P4 corresponding to n2 = 2 are the values of P2 and P4 in the present embodiment.

The reference microorganism is used as a target microbial population that contains only one target microorganism, and the characteristic sequencing fragment of the target microorganism obtained from the calculation is the characteristic sequencing fragment of the reference microorganism. The number of characteristic fragments of the characteristic region of the reference microorganism is shown in Tables 1 and 2.

If the probability of the characteristic sequencing fragment of the target microbial population P5 ≥ α5, determine that the target microbial population is present in the sample to be tested; if the probability of the characteristic sequencing fragment of the target microbial population P5 < α5, determine that the target microbial population is not present in the sample to be tested, wherein α5 is a probability guarantee. In this embodiment, α5 has a value of 99.99%. P5=1-BINOM.DIST(S1,S1,P1,FALSE), S1 is the median of the number of the characteristic sequencing fragments of the target microbial population of all the characteristic regions of the target microbial population; in this embodiment, the number of the second characteristic sequencing fragment of the target microbial population is the median of the number of characteristic sequencing fragments of all the target microbial populations. The value of S1 in the present embodiment is shown in Table 1 and Table 2, and the values of S1 and P1 in this embodiment are substituted into the calculation formula of P5 so as to obtain P5 ≥ α5. Therefore, in this embodiment, the target microbial population exists in the sample to be tested; FALSE is the parameter value, and the BINOM.DIST function returns the probability of the binomial distribution.

If the probability of the characteristic sequencing fragment of the target microorganism P6 ≥ α6, determine that the target microorganism is present in the sample to be tested; if the probability of the characteristic sequencing fragment of the target microorganism P6 < α6, determine that the target microorganism is not present in the sample to be tested; and wherein α6 is a probability guarantee. In this embodiment, α6 has a value of 99.99%. P6=1-BINOM.DIST (S3,S3,P2,FALSE), BINOM.DIST function returns the probability of the binomial distribution. S3 is the median of the number of characteristic sequencing fragments of the target microorganism of all the characteristic regions of the target microorganism. In the present embodiment, the number of the second characteristic sequencing fragment of the target microorganism is the median of the number of all characteristic sequencing fragments of the target microorganism. The corresponding value of S3 is shown in Table 1 and Table 2. The value of S3 and the value of P2 in this embodiment are substituted into the calculation formula of P6 to obtain P6 ≥ α6. Therefore, in this embodiment, it is determined that the target microorganism is present in the sample to be tested.

In addition, both α5 and α6 are determined according to actual needs. The values of α5 and α6 can be the same or different, and the difference therebetween depends on the actual needs. When a certain microorganism needs be strictly controlled, the values of α5 and α6 are relatively large. In an opposite case, the values of α5 and α6 are both small. In addition, the values in the embodiment of the present invention follows the same rule.

The quantitative analysis method is as follows: the amount of the target microbial population M1=Mr×S1/S2, wherein Mr is the amount of the reference microorganism added to the sample to be tested. In this embodiment, the value of Mr is shown in Table 2. S2 is the median of the number of the characteristic sequencing fragments of the reference microorganism of all the characteristic regions of the reference microorganism. In this embodiment, the number of the second sequencing fragment of the reference microorganism is the median of the number of characteristic sequencing fragments of all reference microorganisms, and the corresponding value of S2 is shown in Table 1 and Table 2. The value of S1 obtained by the qualitative analysis and the foregoing parameter values are substituted into the calculation formula of M1, and the M1 value is calculated, that is, the amount of microorganisms in the target microbial population in the sample to be tested is M1=2871226.

The confidence interval of the amount of the target microorganism is [M11, M12], and M11 and M12 are respectively the lower limit and the upper limit of the confidence interval of the M1 value. M11=M1×(1-S4/S1), M12=M1×(1+S5/S1), wherein S4 is the number of the false positive characteristic sequencing fragments of the target microbial population and S4=CRITBINOM(nS,P1,α9), S5 is the number of the false negative characteristic sequencing fragments of the target microbial population and S5=CRITBINOM(S1,P3,α9), wherein α9 is a probability guarantee. In this embodiment, the value of α9 is 99.50%, and the CRITBINOM function returns the minimum value that causes the cumulative binomial distribution to be greater than or equal to the critical value; nS is the number of the high throughput sequencing fragments of the non-characterized region amplified by the multiplex amplification primers of the characteristic region of the target microbial population for calculating S1, that is, it refers to the high throughput sequencing fragments amplified by the multiplex primers except the characteristic sequencing fragment of the target microorganism. In this embodiment, nS is the number of the high throughput sequencing fragments of the non-characteristic region generated in the amplification by the multiplex amplification primer of the second characteristic region in the target microbial population. In this embodiment, the value of nS is shown in Table 2. The value of nS and the value of P1 are substituted into the formula of S4 to obtain the value of S4, and the value S1 and the value of P3 in the present embodiment are substituted into the formula of S5 to obtain the value of S5. After obtaining the values of all the parameters in the M11 and M12 formulas, the values of M11 and M12 in the present example can be obtained by calculation, so as to obtain the confidence interval of M1, that is, the confidence interval of the amount of the target microbial population is [2871226, 2871455].

The amount of the target microorganism M2 = M1 × S3/S1, and the values of M1, S3 and S1 were substituted into the foregoing formula to obtain the amount of the target microorganism M2 = 2534075.

The confidence interval of the amount of the target microorganism is [M21, M22], and M21 and M22 are respectively the lower limit and the upper limit of the confidence interval of the M2 value; M21=M2×(1-S6/S3), M22=M2×(1+S7/S3); wherein S6 is the number of the false positive characteristic sequencing fragments of the target microorganism and S6=CRITBINOM(S1,P2,α10), S7 is the number of the false negative characteristic sequencing fragments of the target microorganism and S7=CRITBINOM (S3,P4,α10), where α10 is a probability guarantee; the CRITBINOM function returns a minimum value that makes a cumulative binomial distribution greater than or equal to a critical value. In the present embodiment, the value of α10 is 99.50%, and the values of S1 and S3 and the values of P2 and P4 in this embodiment are substituted into the calculation formulas of S6 and S7, and the values of S6 and S7 are calculated. Further, the values of S6, S7, M1, and S3 are substituted into the calculation formulas of M21 and M22, and the values of M21 and M22 are calculated, and the obtained confidence interval of the amount of the target microorganism is [2534067, 2539614].

**Table 2. Parameters and calculation mechanism of microbial qualitative and quantitative analysis of this example**

| | | | | |
|---|---|---|---|---|
| Basic parameters | nS | Mr | S1 | S2 |
| | 47525 | 2000000 | 325564 | 226777 |
| | S3 | E1 | E2 | E |
| | 287335 | 0.01 | 0.01 | =SUM(C4:D4) |
| Estimate of the parameters for target microbial population qualitative and quantitative detection | m1 | n1 | L1 (bp) | P1 |
| | 33 | 13 | 195 | =BINOM.DIST(C6,B6, 1-E4,TRUE) |
| | P3 | P5 | α9 | S4 |
| | =1-BINOMDIS T(C6,D6,E4,TRUE) | =1-BINOMDIS T(D2,D2,E6,FALS E) | 0.995 | =CRITBINOM(B2,E6,D8 ) |
| | S5 | M1 | M11 | M12 |
| | =CRITBINOM(D2,B8, D8) | =C2*D2/E2 | =C10*(( 1-E8/D2) | =C10*(1+B10/D2) |
| Estimate of the parameters for target | m2 | n2 | L2 (bp) | P2 |
| | 7 | 2 | 13 | =BINOM.DIST(C12,B12, 1-E4,TRUE) |
| | P4 | P6 | α10 | S6 |
| microorganism qualitative and quantitative detection | =1-BINOMDIS T(C12,D12,E4, TRUE) | =1-BINOMDIS T(B4,B4,E12,F ALSE) | 0.995 | =CRITBINOM(D2,E12,D 14) |
| | S7 | M2 | M21 | M22 |
| | =CRITBINOM( B4,B14,D14) | =C10*B4/D2 | =C16*(1 -E14/B4) | =C16*(1+B16/B4) |

### Example 2: Identification of human feces microorganisms

The sample to be tested in this embodiment is human feces, and is taken from a patient having an intestinal disease as diagnosed by a doctor, and the detection of the microorganism in the patient's feces is a basis for providing a treatment plan. This embodiment is similar to the method of the first embodiment, and the methods, parameters, and results that are not mentioned herein are the same as those of the first embodiment, and therefore, will not be repeated.

Step I - Determine a target microbial population, a target microorganism and a non-target organism in the sample to be tested, and a reference microorganism not present in the sample to be tested.

The purpose of this example is to identify *Salmonella enterica* in the sample to be tested, its Latin name is *Salmonellaenterica*, and in the NCBI (National center for biotechnology information), the *Salmonella enterica* of the reference genome has a total of 33 physiological races (up to the date of June 2, 2015); for more information, please see http://www.ncbi.nlm.nih.gov/genome/genomegroups/152. These physiological races constitute the target microbial population of this embodiment. Among these physiological races, *Salmonella enterica subsp.houtenae* str.ATCC BAA-1581 is highly pathogenic and serves as a target microorganism of the present example.

Step II - Obtaining a characteristic region of the target microbial population, a characteristic region of the target microorganism and a characteristic region of the reference microorganism according to the reference genomic sequence of the target microbial population, the reference genomic sequence of the target microorganism, the reference genomic sequence of the reference microorganism and the reference genomic sequence of the non-target organism. The characteristic region related information finally obtained in this embodiment is shown in Table 3.

**Table 3. Related information of the primers provided in the second embodiment of the present invention**

| Characteristic region | | Start position | End position | Length (L) | Upstream primer | Downstream primer | M1 value | M2 value | Number of characteristic sequencing fragments | |
|---|---|---|---|---|---|---|---|---|---|---|
| | | | | | | | | | Target microbial population | Target microorganism |
| Target microbial population and target microorganism | 1 | 2288 074 | 2288 276 | 20 3 | As shown in SEQ ID No: 13 | As shown in SEQ ID No: 14 | 17 | 7 | 200 350 | 989 9 |
| | 2 | 2986 262 | 2986 411 | 20 3 | As shown in SEQ ID No: 15 | As shown in SEQ ID No: 16 | 68 | 4 | 245 278 | 111 222 |
| | 3 | 4040 443 | 4040 630 | 20 3 | As shown in SEQ ID No: 17 | As shown in SEQ ID No: 18 | 5 | 4 | 354 236 | 150 232 |
| Reference microorganism | 1 | The same as Table 1 | | | | | | | 78679 | |
| | 2 | | | | | | | | 124423 | |
| | 3 | | | | | | | | 153325 | |

Step IV - Adding the reference microorganism to the sample to be tested so as to obtain a mixed sample, and the specific method is as follows:
The method for obtaining the mixed sample in the present embodiment is as follows: 0.2 mL of bacterial solution of the reference microorganism with a concentration of 2 OD (OD is the maximum absorbance value of the bacterial solution) is loaded in a 1.5 mL centrifuge tube, which is dried by vacuum-frozen centrifugation, and then added to 100 mg of the sample to be tested, mix well, so as to obtain a mixed sample of the sample to be tested and the reference microorganism. The amount of the reference microorganism added to the mixed sample is counted by an approach of blood plate counting, and the result is shown in Table 4.

Step V - Extracting the nucleic acid from the mixed sample, and the specific method is as follows:
In this embodiment, the sample to be tested is feces and its nucleic acid content is low. Therefore, an exogenous nucleic acid, that is, 1 µg of an ERCC-00014 gene designed by the external RNA control association, is added to the mixed sample. The nucleic acid of the obtained mixed sample is extracted using a fecal DNA kit (manufacturing company: American MP Company, Cat. No.: 116570200, product English name: FastDNA SPIN kit for feces) according to the method provided in the instructions.

Step VI - Carrying out an amplification reaction using the mixed multiplex amplification primer and the nucleic acid of the mixed sample to obtain an amplification product, and the specific method is the same as that in the first embodiment.

Step VII - Carrying out a high throughput sequencing process with the amplification product, so as to obtain high throughput sequencing fragments, and the specific method is the same as in the first embodiment.

Step VIII - Carrying out qualitative and quantitative analysis of the target microbial population and the target microorganism according to the high throughput sequencing fragments, and the specific method is as follows:
The specific parameters of this embodiment of the present invention and the calculation mechanism thereof are shown in Table 4. The analysis result of the present embodiment is as follows: the target microbial population and the target microorganism are present in the sample to be tested, where the amount of the microorganism in the target microbial population is M1=3942647, the confidence interval is [3942647, 3943113]; the amount of the target microorganism M2=1787805, and the confidence interval is [1777581, 1788849].

**Table 4. Parameters and calculation mechanism of microbial qualitative and quantitative analysis of this example**

| | | | | |
|---|---|---|---|---|
| Basic parameters | nS | Mr | S1 | S2 |
| | 30755 | 2000000 | 245278 | 124423 |
| | S3 | E1 | E2 | E |
| | 111222 | 0.01 | 0.01 | =SUM(C4:D4) |
| Estimate of the parameters for target microbial population qualitative and quantitative detection | m1 | n1 | L1 (bp) | P1 |
| | 68 | 13 | 203 | =BINOM.DIST(C6,B6, 1-E4,TRUE) |
| | P3 | P5 | α9 | S4 |
| | =1-BINOMDIS T(C6,D6,E4,TRUE) | =1-BINOMDIS T(D2,D2,E6,FAL SE) | 0.995 | =CRITBINOM(B2,E6,D 8) |
| | S5 | M1 | M11 | M12 |
| | =CRITBINOM(D2,B8, D8) | =C2*D2/E2 | =C10*(( 1-E8/D2) | =C10*(1+B10/D2) |
| Estimate of the parameters for target microorganis m qualitative and quantitative detection | m2 | n2 | L2 (bp) | P2 |
| | 4 | 2 | 8 | =BINOM.DIST(C12,B12 ,1-E4,TRUE) |
| | P4 | P6 | α10 | S6 |
| | =1-BINOMDIS T(C12,D12,E4, TRUE) | =1-BINOMDIS T(B4,B4,E12,F ALSE) | 0.995 | =CRITBINOM(D2,E12, D14) |
| | S7 | M2 | M21 | M22 |
| | =CRITBINOM(B4,B14,D14) | =C10*B4/D2 | =C16*(1 - E14/B4) | =C16*(1+B16/B4) |

The detection method provided by the embodiments of the present invention can be applied in various areas of medicine. In different applications, the microbial nucleic acid separation methods are slightly different. For example, blood and feces have different genomic extraction kits, and they need to be operated according to their respective operation instructions. The other steps are basically the same except for the nucleic acid separation method. Therefore, the detection method provided by the embodiments of the present invention is very versatile. The present invention changes the existing method which has certain drawbacks, such as it can only detect a few microorganisms at a time, can only distinguish microorganisms into species, is quantitatively inaccurate, has no probabilistic guarantee of detection results, requires pre-culture, a long detection period, and in the case that some microorganisms cannot be cultured and thus cannot be detected, has quantitative distortion due to different microbial culturability, has rough quantification and many other problems. The present invention provides a comprehensive, fast and precise qualitative and quantitative detection method for human microbiological detection, and provides fast, accurate and comprehensive data support for medical diagnosis.

## Claims

1. A method for qualitative and quantitative detection of a microorganism in a human body, **characterized in that** the method comprises:
determining a target microbial population, a target microorganism and a non-target organism in a sample to be tested, and a reference microorganism not present in the sample to be tested, wherein the sample to be tested is a human tissue, body fluid and feces;
obtaining a characteristic region of the target microbial population, a characteristic region of the target microorganism and a characteristic region of the reference microorganism according to the reference genomic sequences of the target microbial population, the target microorganism, the reference microorganism and the non-target organism;
preparing a first multiplex amplification primer for amplifying the characteristic region of the target microbial population, a second multiplex amplification primer for amplifying the characteristic region of the target microorganism, and a third multiplex amplification primer for amplifying the characteristic region of the reference microorganism, and mixing the first multiplex amplification primer, the second multiplex amplification primer and the third multiplex amplification primer so as to obtain mixed multiplex amplification primers;
adding the reference microorganism to the sample to be tested so as to obtain a mixed sample;
extracting the nucleic acid of the mixed sample;
carrying out an amplification reaction using the mixed multiplex amplification primers and the nucleic acid of the mixed sample, so as to obtain an amplification product;
carrying out a high throughput sequencing using the amplification product, so as to obtain a high throughput sequencing fragment; and
carrying out qualitative and quantitative analysis with the target microbial population and the target microorganism.

2. The method according to claim 1, **characterized in that** the number of the target microbial population is ≥ 1, and each target microbial population comprises ≥ 0 types of the target microorganism;
the target microorganism is at least one selected from the group consisting of bacterium, virus, fungus, actinomycetes, rickettsia, mycoplasma, chlamydia, spirochete and protozoa; and
the reference microorganism is at least one selected from the group consisting of bacterium, virus, fungus, actinomycetes, rickettsia, mycoplasma, chlamydia, spirochete and protozoa.

3. The method according to claim 1, **characterized in that** the step of determining a non-target organism in a sample to be tested is carried out by a method that comprises: determining the non-target organism to be all organisms except the target microbial population, if the characteristic region of the target microbial population is obtained, the non-target organism referring to all organisms except the target microbial population; if the characteristic region of the target microbial population is not obtained, the non-target organism referring to the organisms other than the target microbial population in the mixed sample.

4. The method according to claim 1, **characterized in that** the characteristic region of the target microbial population is a nucleic acid sequence on a reference genome of the microorganism within the target microbial population; sequences on both sides of the characteristic region of the target microbial population are a single sequence in the reference genome; the sequences on both sides of the characteristic region of the target microbial population are conservative among different microorganisms in the target microbial population; and the distinguishing degree of the characteristic region of the target microbial population is ≥ 3;
the characteristic region of the target microorganism is homologous to the characteristic region of the target microbial population; the characteristic region of the target microorganism has an m2 value ≥ 2, wherein the m2 value is a minimum value of the number of different bases between the characteristic region of the target microorganism and the microorganisms other than the target microorganism within the target microbial population;
the characteristic region of the reference microorganism is a nucleic acid sequence in the reference genome of the reference microorganism; sequences on both sides of the characteristic region of the reference microorganism are a single sequence in the reference genome of the reference microorganism; the sequences on both sides of the characteristic region do not have homology in organisms other than the reference microorganism.

5. The method according to claim 4, **characterized in that** the distinguishing degree refers to a minimum value of the number of different bases between a characteristic region of any target microbial population and any non-characteristic region amplified by the same mixed multiplex amplification primers, wherein the non-characteristic region is an amplification product of the mixed multiplex amplification primers with the nucleic acid of the mixed sample as a template, and the non-characteristic region is not a characteristic region of the target microbial population; if the non-characteristic region is absent, the distinguishing degree is 3 × L1/4, wherein L1 is the length of a nucleic acid sequence of the characteristic region of the target microbial population.

6. The method according to claim 1, **characterized in that** the method further comprises:
when extracting a nucleic acid of the mixed sample, if the content of the nucleic acid in the sample to be tested is too low, in the process of extracting the nucleic acid of the mixed sample, adding an exogenous nucleic acid that cannot be amplified by the mixed multiplex amplification primers.

7. The method according to claim 1, **characterized in that** a qualitative analysis method of the target microbial population and the target microorganism is as follows:
comparing the high throughput sequencing fragment with the characteristic region of each target microbial population, and when the number of different bases is ≤ n1, the comparison is successful, and the corresponding high throughput sequencing fragment is the characteristic region of the target microbial population, wherein n1 is a maximum error-tolerant number of bases of a characteristic sequencing fragment of the target microbial population; and if the characteristic region of the target microbial population of a successful comparison ≥ 1, determining that the high throughput sequencing fragment is the characteristic sequencing fragment of the target microbial population;
comparing the characteristic region of the target microorganism with the characteristic region of each of the homologous target microbial populations, and extracting the different bases from the characteristic region of the target microorganism to form a standard genotype of the target microorganism; extracting the bases corresponding to the standard genotype of the target microorganism from the characteristic sequencing fragment of the target microbial population to form a test genotype of the target microorganism; if the number of different bases between the test genotype of the target microorganism and the standard genotype of the target microorganism ≤ n2, wherein n2 is a maximum error-tolerant number of bases of the characteristic sequencing fragment of the target microorganism, the high throughput sequencing fragment where the test genotype of the target microorganism is located is a characteristic sequencing fragment of the target microorganism;
calculating the obtained characteristic sequencing fragment of the target microorganism with the reference microorganism as the target microbial population that contains only one target microorganism, which is the characteristic sequencing fragment of the reference microorganism;
if the probability of the characteristic sequencing fragment of the target microbial population P5 ≥ α5, determining that the target microbial population is present in the sample to be tested, wherein α5 is a probability guarantee; if the probability of the characteristic sequencing fragment of the target microbial population P5 < α5, determining that the target microbial population is not present in the sample to be tested;
if the probability of the characteristic sequencing fragment of the target microorganism P6 ≥ α6, determining that the target microorganism is present in the sample to be tested, wherein α6 is a probability guarantee; if the probability of the characteristic sequencing fragment of the target microorganism P6 < α6, determining that the target microorganism is not present in the sample to be tested;
n1 allowing P1 ≤ α1, and P3 ≤ α3, wherein P1 is the probability of a false positive generated when one high throughput sequencing fragment that is not a characteristic sequencing fragment of the target microbial population is misidentified as a characteristic sequencing fragment of the target microbial population; P3 is the probability of a false negative generated when one high throughput sequencing fragment that is a characteristic sequencing fragment of the target microbial population is misidentified as not a characteristic sequencing fragment of the target microbial population; wherein α1 and α3 are the thresholds for respective determinations;
n2 allowing P2 ≤ α2, and P4 ≤ α4, wherein P2 is the probability of a false positive generated when one high throughput sequencing fragment that is not a characteristic sequencing fragment of the target microorganism is misidentified as a characteristic sequencing fragment of the target microorganism; P4 is the probability of a false negative generated when one high throughput sequencing fragment that is a characteristic sequencing fragment of the target microorganism is misidentified as not a characteristic sequencing fragment of the target microorganism; wherein α2 and α4 are the thresholds for respective determinations;
P5=1-BINOM.DIST(S1,S1,P1,FALSE), P6=1-BINOM.DIST(S3,S3,P2,FALSE), S1 is the median of the number of the characteristic sequencing fragments of the target microbial population of all the characteristic regions of the target microbial population; S3 is the median of the number of the characteristic sequencing fragments of the target microorganism of all the characteristic regions of the target microorganism; FALSE is a parameter value; BINOM.DIST function returns the probability of a binomial distribution.

8. The method according to claim 7, **characterized in that** a quantitative analysis method of the target microbial population and the target microorganism is as follows:
the amount of the target microbial population M1=Mr×S1/S2, and the confidence interval of the amount of the target microbial population is [M11, M12], wherein Mr is the amount of the reference microorganism added to the sample to be tested; S2 is the median of the number of the characteristic sequencing fragments of the reference microorganism of all the characteristic regions of the reference microorganism; M11 and M12 are respectively the lower limit and the upper limit of the confidence interval of the M1 value;
the amount of the target microorganism M2=M1×S3/S1, the confidence interval of the amount of the target microorganism is [M21, M22], and M21 and M22 are respectively the lower limit and the upper limit of the confidence interval of the M2 value;
M11=M1×(1-S4/S1), M12=M1×(1+S5/S1), M21=M2×(1-S6/S3), M22=M2×(1+S7/S3); wherein S4 is the number of the false positive characteristic sequencing fragments of the target microbial population and S4=CRITBINOM(nS,P1,α9), wherein nS is the number of the high throughput sequencing fragments of the non-characterized region amplified by the multiplex amplification primers of the characteristic region of the target microbial population for calculating S1; S5 is the number of the false negative characteristic sequencing fragments of the target microbial population and S5=CRITBINOM(S1,P3,α9), wherein α9 is a probability guarantee; S6 is the number of the false positive characteristic sequencing fragments of the target microorganism and S6=CRITBINOM (S1,P2,α10); S7 is the number of the false negative characteristic sequencing fragments of the target microorganism and S7= CRITBINOM(S3,P4,α10), where α10 is a probability guarantee; the CRITBINOM function returns a minimum value that makes a cumulative binomial distribution greater than or equal to a critical value.

9. The method according to claim 8, **characterized in that** P1=BINOM.DIST(n1,m1,1-E,TRUE), P2=BINOM.DIST(n2,m2,1-E,TRUE), P3=1-BINOM.DIST(n1,L1,E,TRUE), and P4=1-BINOM.DIST(n2,L2,E,TRUE), wherein m1 is the distinguishing degree; m2 is a minimum value of the different bases between the characteristic region of the target microorganism and the other microorganisms within the target microbial population; L1 is the length of the characteristic region of the target microbial population; L2 is the length of the standard genotype of the target microorganism; and E is a base error rate.
